# EUROPÄISCHE PATENTSCHRIFT

(11) **EP 2 618 759 B1**
(45) Veröffentlichungstag und Bekanntmachung des Hinweises auf die Patenterteilung: **21.10.2015**
(21) Anmeldenummer: 11767616.3
(22) Anmeldetag: 10.08.2011
(51) Int. Cl.: A61B 17/88, B01F 7/00, B01F 15/00, B01F 15/02, B01F 13/00, B01F 7/16, B29B 7/10

(54) **MISCHVORRICHTUNG FÜR PREPACK-VAKUUM-ZEMENTIERSYSTEM, VAKUUM-ZEMENTIERSYSTEM UND VERFAHREN**
MIXING DEVICE FOR PREPACK VACUUM CEMENTING SYSTEM, VACUUM CEMENTING SYSTEM AND METHOD
DISPOSITIF DE MÉLANGE POUR SYSTÈME DE CIMENTATION SOUS VIDE À PRÉ-EMBALLAGE, SYSTÈME DE CIMENTATION SOUS VIDE ET PROCÉDÉ

(30) Priorität: 22.09.2010 DE 102010046055
(43) Veröffentlichungstag der Anmeldung: 31.07.2013
(73) Patentinhaber: Heraeus Medical GmbH, 61273 Wehrheim (DE)
(72) Erfinder: VOGT, Sebastian, 99092 Erfurt (DE); BUECHNER, Hubert, 90491 Nuernberg (DE)
(74) Vertreter: Sterzel, Roland
(86) Internationale Anmeldenummer: PCT/EP2011/003992
(87) Internationale Veröffentlichungsnummer: WO 2012/038002

(56) Entgegenhaltungen:
- WO-A1-99/67015
- DE-A1- 2 825 230
- DE-B3-102009 031 178
- US-A- 3 140 078
- US-A1- 2004 196 735
- US-A1- 2005 155 901

## Beschreibung

Die Erfindung betrifft eine Mischvorrichtung entsprechend Anspruch 1 zum Mischen eines Mischguts, insbesondere eines medizinischen Zements, umfassend zumindest eine Kartusche und einen Verschluss, wobei die zumindest eine Kartusche eine erste Komponente des Mischguts beinhaltet und einen Kartuschenkopf umfasst, in dem wenigstens eine Belüftungsöffnung angeordnet ist und der Verschluss derart am Kartuschenkopf angeordnet ist, dass die wenigstens eine Belüftungsöffnung im Kartuschenkopf durch den Verschluss verschließbar und zu öffnen ist.

Die Erfindung betrifft auch ein Vakuumzementiersystem entsprechend Anspruch 17 mit einer solchen Mischvorrichtung und ein Verfahren entsprechend Anspruch 18 zur Verwendung einer solchen Mischvorrichtung oder eines solchen Vakuumzementiersystems.

Dabei wird eine Vorrichtung zum Mischen eines Zements aus einem Monomer und einem Zementpulver bereitgestellt.

Polymethylmethacrylat-Knochenzemente (PMMA-Knochenzemente) sind seit Jahrzehnten bekannt und gehen auf die grundlegenden Arbeiten von Sir Charnley zurück (Charnley, J.: Anchorage of the femoral head prosthesis of the shaft of the femur. J. Bone Joint Surg. 42 (1960) 28-30.). Der Grundaufbau der PMMA-Knochenzemente ist seit dem prinzipiell gleich geblieben. PMMA-Knochenzemente bestehen in der Regel aus einer flüssigen Monomerkomponente und einer Pulverkomponente. Die Monomerkomponente enthält im Allgemeinen das Monomer Methylmethacrylat und einen darin gelösten Aktivator (N,N-Dimethyl-p-toluidin). Die Pulverkomponente besteht aus einem oder mehreren Polymeren, die auf Basis von Methylmethacrylat und Comonomeren, wie Styren, Methylacrylat oder ähnlichen Monomeren, durch Polymerisation, vorzugsweise Suspensionspolymerisation, hergestellt sind, einem Röntgenopaker und dem Initiator Dibenzoylperoxid. Beim Vermischen der Pulverkomponente mit der Monomerkomponente entsteht durch Quellung der Polymere der Pulverkomponente im Methylmethacrylat ein plastisch verformbarer Teig. Bei dem Vermischen der Pulverkomponente mit der Moriomerkomponente reagiert der Aktivator N,N-Dimethyl-p-Toluidin mit Dibenzoylperoxid unter Bildung von Radikalen. Die gebildeten Radikale initiieren die radikalische Polymerisation des Methylmethacrylates. Mit fortschreitender Polymerisation des Methylmethacrylates erhöht sich die Viskosität des Zementteigs bis dieser erstarrt.

PMMA-Knochenzemente können in geeigneten Mischbechern mit Hilfe von Spateln durch Vermischen des Zementpulvers mit der Monomerflüssigkeit vermischt werden. Nachteilig ist an dieser Vorgehensweise, dass Lufteinschlüsse im gebildeten Zementteig vorhanden sein können, die später eine Destabilisierung des Knochenzementes verursachen können. Aus diesem Grund wird das Vermischen von Knochenzementpulver mit der Monomerflüssigkeit in Vakuummischsystemen bevorzugt, weil durch Mischen im Vakuum Lufteinschlüsse aus dem Zementteig weitgehend entfernt werden und damit eine optimale Zementqualität erreicht wird (Breusch SJ et al.: Der Stand der Zementiertechnik in Deutschland. Z Orthop. 1999, 137: 101-07). Im Vakuum gemischte Knochenzemente haben eine deutlich verringerte Porosität und zeigen daher verbesserte mechanische Eigenschaften. Es ist eine Vielzahl an Vakuumzementiersystemen bekannt, die beispielsweise in den folgenden Patentdokumenten beschrieben sind: US 5,624,184, US 4,671,263, US 4,973,168, US 5,100,241, WO 99/67015 A1, EP 1 020 167 A2, US 5,586,821, EP 1 016 452 A2, DE 36 40 279 A1, WO 94/26403 A1, EP 0 692 229 A1, EP 1 005 901 A2, US 5,344,232.

Vakuumzementiersysteme werden beim Mischen des PMMA-Knochenzements unter Vakuum gesetzt, um Lufteinschlüsse aus dem Zementteig zu entfernen. Es soll dadurch ein möglichst homogener, weitgehend blasenfreier Zementteig gebildet werden.

Eine Weiterentwicklung stellen Zementiersysteme dar, in denen sowohl das Zementpulver als auch die Monomerflüssigkeit bereits in separaten Kompartimenten der Mischsysteme verpackt sind und erst unmittelbar vor der Zementapplikation im Zementiersystem miteinander vermischt werden (US 5,997,544, EP 0 692 229 A1, US 6,709,149 B1). Problematisch ist bei diesen Systemen die Überführung der Monomerflüssigkeit in das Zementpulver und die vollständige Vermischung dieser beiden Komponenten, damit eine homogener Zementteig erhalten wird, der insbesondere keine Nester an nicht von der Monomerflüssigkeit benetztem Zementpulver enthalten darf. Bei dem gegenwärtig in Europa auf dem Markt befindlichen *Full-Prepack-*Mischsystem Optipac™ (Biomet Switzerland) wird durch einfache Röhren, die seitlich im unteren Teil der Kartusche angebracht sind, die die Kartuschenwand durchstoßen, die Monomerflüssigkeit ungefähr in der Mitte des Zementpulvers durch Einwirkung von Vakuum aus Aluminiumverbundbeuteln in das Zementpulver eingesaugt. Aluminiumverbundbeutel werden zum Öffnen manuell gegen die Röhren bewegt, wobei die Röhren die Wandung der Beutel durchstechen.

Aluminiumverbundbeutel sind für die Verpackung und Lagerung von Monomerflüssigkeit erst seit wenigen Jahren bekannt. Sehr gute Erfahrungen hinsichtlich der Lagerfähigkeit von Monomerflüssigkeit liegen mit Glasampullen vor. Glasampullen werden seit Jahrzehnten für konventionelle Polymethylmethacrylat-Knochenzemente mit gutem Erfolg eingesetzt. Glasampullen haben neben der perfekten Dichtigkeit auch den Vorteil, dass sie in großen Stückzahlen zu niedrigen Preisen gefertigt werden können. Daher ist es sinnvoll, Glasampullen zur Verpackung und Lagerung von Monomerflüssigkeit in Prepack-Vakuumzementiersystemen einzusetzen.

Bei der Verwendung von Kartuschensystemen für sterile pastöse Medizinprodukte ist es notwendig, dass nicht nur die Pasten sondern natürlich auch die Kartuschen und das sekundäre Packmittel in steriler Form dem medizinischen Anwender zur Verfügung gestellt werden. So können zum Beispiel nach aseptischer Befüllung der zuvor sterilisierten Kartuschen diese direkt in ein steriles Packmittel überführt werden. Weiterhin kann es bei bestimmten Produkten sinnvoll sein, die Oberflächen der befüllten Kartuschen zusammen mit den Packmitteln nach erfolgter Verpackung zu sterilisieren. Neben der Gammasterilisation, die bei polymerisierbaren Pastensystemen nicht angewendet werden kann, besteht die Möglichkeit mit dem Gas Ethylenoxid zu sterilisieren.

In der DE 195 32 015 A1 ist eine Vorrichtung zum Mischen und Ausbringen von Mehrkomponentenprodukten beschrieben. An der Außenseite der Zementkartusche ist eine Lagerbuchse ausgebildet, um die sich drehbar ein Ampullenhalter bewegen kann. Der Kopf der Ampulle befindet sich im Inneren der Lagerbuchse. Bei der Drehung des Ampullenhalters um die Lagerbuchse wird der Ampullenkopf vom Ampullenkörper abgeschert. Dann kann die Flüssigkeit aus der Ampulle in die Kartusche durch eine Öffnung in der Kartuschenwand überführt werden. Dort ist ein Mischelement angeordnet, mit dem ein Zementpulver mit der Flüssigkeit aus der Ampulle gemischt werden kann.

In der WO 97/18031 A1 wird eine Vorrichtung vorgeschlagen, bei der eine Ampulle am Boden durchstoßen wird und die Monomerflüssigkeit danach durch einen hohlen Mischstab in die Zementkartusche fließen kann.

Ein System zum Mischen eines medizinischen Zements unter Vakuum ist aus der EP 1 031 333 A1 bekannt. Bei diesem System wird durch eine Bewegung des Mischstabs gegen eine keilförmige Vorrichtung im Kartuschenkopf der Ampullenkopf schräg gegen die Ampullenachse bewegt, wobei der Ampullenkopf vom Ampullenkörper abgeschert wird. Im Inneren eines Mischraums wird eine Durchmischung des Zements mit einer Mischvorrichtung erreicht.

Die DE 10 2009 031 178 B3 beschreibt ein Zementiersystem, bei dem ein separater Sterilisationskolben separat zu einem Dichtungskolben und einem Mischkolben bewegbar ist. Der Dichtungskolben und der Sterilisationskolben bilden dabei ein gemeinsames kolbensystem.

Aus der DE 10 2007 061 696 B4 ist eine Mischvorrichtung bekannt, die ein Vakuumzementiersystem beschreibt. Die Mischvorrichtung umfasst einen Drehverschluss zum Abschließen eines Mischzylinders. Im geschlossenen Zustand können die zu mischenden Komponenten im Mischzylinder mit einer Mischscheibe gemischt werden. Danach wird das Mischgut durch ein Austragsrohr aus dem Vakuumzementiersystem ausgetragen. Nachteilig ist hieran, dass der Drehverschluss abgenommen beziehungsweise angehoben werden muss, um das Vakuumzementiersystem zu öffnen und so einen Gasdurchlass zum Sterilisieren des Inneren des Vakuumzementiersystems zu ermöglichen. Zudem ist im späteren Verlauf der Anwendung des Vakuumzementiersystems das Vakuum schwer anzulegen, da die Evakuierung durch das Zementpulver erfolgen muss.

Der Erfindung liegt die Aufgabe zu Grunde, eine Vorrichtung und ein Verfahren zu entwickeln, mit denen eine einfachere Handhabung eines Vakuumzementiersystems ermöglicht wird. Zudem sollen das Sterilisieren, das Mischen der Komponenten und das Anlegen von Vakuum möglichst leicht durchführbar sein.

Diese Aufgabe wird durch eine Mischvorrichtung entsprechend Anspruch 1 gelöst.

Dabei kann vorgesehen sein, dass die Verbindung ein Rohr oder ein Schlauch ist, wobei vorzugsweise zwischen der Verbindung und dem Behälter ein Ventil angeordnet ist.

Auch kann vorgesehen sein, dass die Verbindung in dem Kartuschenkopf entlang ihrer Symmetrieachse verschiebbar ist.

Eine Weiterbildung der Erfindung sieht vor, dass zwischen der wenigstens einen Belüftungsöffnung im Kartuschenkopf und dem mit der ersten Komponente gefüllten Inneren der Kartusche ein Porenfilter angeordnet ist.

Der Filter, das Sieb oder der Porenfilter dient dazu, ein Eindringen der ersten Komponente (zum Beispiel eines Pulvers) in den Bereich des Kartuschenkopfs zu verhindern.

Erfindungsgemäße Mischvorrichtungen können sich auch dadurch auszeichnen, dass die Mischvorrichtung ein Austragsrohr oder eine Befestigungseinrichtung für ein Austragsrohr umfasst, wobei das Austragsrohr vorzugsweise durch einen entfernbaren Austragsrohrverschluss verschließbar ist

Ferner kann vorgesehen sein, dass die Verbindung einen seitlichen Auslass im Bereich des Endes der Verbindung umfasst, das ins Innere der Kartusche reicht.

Auch kann vorgesehen sein, dass die Verbindung zwischen ihrem Auslass ins Innere der Kartusche und dem Behälter eine Sollbruchstelle umfasst, vorzugsweise im Bereich des Auslasses.

Erfindungsgemäße Mischvorrichtungen zeichnen sich dadurch aus, dass der Verschluss zwischen dem Kartuschenkopf und dem Filter oder dem Sieb, vorzugsweise dem Porenfilter, angeordnet ist, wobei sich zumindest ein Teil, insbesondere wenigstens ein Griff des Verschlusses, vorzugsweise durch die wenigstens eine Belüftungsöffnung im Kartuschenkopf erstreckt.

Es kann auch vorteilhaft sein, dass zwei Belüftungsöffnungen in dem Kartuschenkopf angeordnet sind, die durch zwei Blätter des Verschlusses verschließbar sind, wobei die Belüftungsöffnungen und die Blätter vorzugsweise als Kreissektoren ausgebildet sind und der Verschluss drehbar ist.

Eine Weiterbildung der Erfindung sieht vor, dass am Verschluss zumindest ein Griff angeordnet ist, vorzugsweise zwei Griffe angeordnet sind, mit denen der Verschluss drehbar oder verschiebbar ist.

Des Weiteren kann vorgesehen sein, dass ein Griffstück mit dem Austragsrohr oder einem Mischstab, der sich durch eine zentrische Öffnung im Kartuschenkopf von außen bis ins Innere der Kartusche erstreckt, verbunden oder verbindbar ist, mit dem das Austragsrohr oder der Mischstab im Inneren der Kartusche drehbar ist.

Dabei kann vorgesehen sein, dass der Austragsrohrverschluss ein Stopfen im Inneren der Austragsrohrs ist, der mit dem Griffstück verbunden ist, mit dem der Stopfen aus dem Austragsrohr entfernbar ist.

Erfindungsgemäße Mischvorrichtungen können sich auch dadurch auszeichnen, dass am Austragsrohr oder am Mischstab wenigstens ein Mischflügel angeordnet ist und das Austragsrohr oder der Mischstab mit dem Mischflügel oder den Mischflügeln drehbar in der Kartusche gelagert ist.

Ferner kann vorgesehen sein, dass im Kartuschenkopf oder in einer Wand der Kartusche ein Vakuumanschluss als Durchführung angeordnet ist, der vorzugsweise einen Ventilverschluss zum Öffnen und Schließen des Vakuumanschluss umfasst.

Eine weitere Ausgestaltung der Erfindung sieht vor, dass im Kartuschenboden ein Förderkolben zum Austreiben des Kartuscheninhalts angeordnet ist.

Besonders bevorzugt ist, dass die erste Komponente ein Pulver, insbesondere ein Zementpulver für einen PMMA-Knochenzement ist, und die zweite Komponente eine Flüssigkeit, insbesondere eine Monomerflüssigkeit, ist.

Die Aufgabe der Erfindung wird auch gelöst durch ein Vakuumzementiersystem entsprechend Anspruch 17.

Die der Erfindung zugrunde liegende Aufgabe wird auch durch ein Verfahren entsprechend Anspruch 18 gelöst.

Dabei sind die folgenden Verfahrensschritte vorgesehen
A) das Innere und der Inhalt der Kartusche wird durch die wenigstens eine geöffnete Belüftungsöffnung im Kartuschenkopf sterilisiert, insbesondere unter Verwendung von Ethylenoxid,
B) die wenigstens eine Belüftungsöffnung im Kartuschenkopf wird durch Drehen oder Verschieben des Verschlusses relativ zum Kartuschenkopf oder durch Drehen oder Verschieben des Kartuschenkopfs relativ zum Verschluss geschlossen,
C) Gase aus dem Inneren der Kartusche werden evakuiert,
D) die zweite Komponente des Behälters wird in die Kartusche geleitet und
E) die erste und die zweite Komponente werden gemischt.

Unter einer Sterilisation des Inneren und insbesondere des Inhalts der Kartusche ist auch eine teilweise und/oder bereichsweise Sterilisation zu verstehen.

Ferner kann vorgesehen sein, dass die Mischung durch ein Austragsrohr appliziert wird.

Schließlich ist vorgesehen dass nachdem die zweite Komponente ins Innere der Kartusche geleitet wurde, die Verbindung zum Behälter getrennt wird und der nicht zentrische Durchgang für die Verbindung zum Behälter durch Drehen oder Verschieben des Verschlusses relativ zum Kartuschenkopf oder umgekehrt geschlossen wird.

Der Erfindung liegt die überraschende Erkenntnis zugrunde, dass eine Mischvorrichtung mit einem drehbaren oder verschiebbaren Verschluss zum Verschließen einer Belüftungsöffnung ganz einfach und kostengünstig aufgebaut werden kann. Die Funktionsweise der Mischvorrichtung ist dabei so einfach, dass sie jederzeit leicht anwendbar ist. Auch können alte Mischvorrichtungen leicht umgerüstet werden. Zudem kann eine Durchführung für die zweite Komponente leicht durch einen leicht modifizierten Verschlussmechanismus geschlossen werden, ohne dass hierfür zusätzliche Bauteile notwendig wären. Das Verschlusssystem ist dabei außerordentlich unanfällig für Störungen, da das Verschieben einer Fläche (eines Blatts des Verschlusses) über eine Öffnung leicht umzusetzen ist.

Das zu Grunde liegende Verschlussprinzip wird beispielsweise dadurch verwirklicht, dass oberhalb einer Porenscheibe (eines Porenfilters) in Richtung eines Vakuumanschlusses eine Abdeckung vorhanden ist, die wenigstens eine Belüftungsöffnung als Fenster aufweist. Darüber ist eine drehbare oder verschiebbare Verschlussscheibe oder ein Verschlussscheibenausschnitt angeordnet, die mindestens ebenfalls eine Belüftungsöffnung aufweist. Die Belüftungsöffnung oder die Belüftungsöffnungen müssen dann über das oder die Belüftungsöffnungen der unteren Scheibe gedreht oder geschoben werden können, so dass ein Gasdurchlass zwischen der Porenscheibe und der Umgebung möglich ist. Durch Verdrehung oder Verschiebung der Verschlussscheibe muss es möglich sein, dass das oder die Belüftungsöffnungen der Verschlussscheibe nicht mit dem oder den Belüftungsöffnungen der unteren Scheibe überlappen, so dass ein Gasaustausch zwischen der Porenscheibe zur Umgebung nur über einen Vakuumanschluss möglich ist.

Der Drehverschluss weist einen oder mehrere Belüftungsöffnungen in Form von Kreissektoren ("Tortenstücken") beziehungsweise Kreissektorabschnitten auf. Der Drehverschluss kann entsprechende Belüftungsöffnungen im Kartuschenkopf abdecken oder offen legen.

Durch Drehung des Verschlusses können die Belüftungsöffnungen geöffnet und verschlossen werden, so dass eine Durchlässigkeit für Ethylenoxid während der Sterilisation gegeben ist und bei der Zementapplikation durch einfache Drehung des Drehverschlusses eine Vakuumdichtigkeit erzielt werden kann.

Der Drehverschluss lässt sich einmal so drehen, dass die Belüftungsöffnungen im Kartuschenkopf für die Ethylenoxid-Sterilisation verschlossen sind und Vakuum gezogen werden kann. Nach Herausziehen des Rohrs kann der Drehverschluss um wenige Grad weiter gedreht werden, so dass auch die Durchführung für das Rohr einmal im Kartuschenkopf und gleichzeitig in der Porenscheibe verschlossen ist. Der Drehverschluss hat zu diesem Zweck an der Unterseite eine plane Fläche, welche die Durchführung in der Porenscheibe abdecken kann.

Die Funktionsweise einer erfindungsgemäßen Mischvorrichtung kann wie folgt beschrieben werden. Zuerst wird der Drehverschluss im Kartuschenkopf so gedreht, dass die Belüftungsöffnungen für die Ethylenoxid-Sterilisation verschlossen sind. Dann wird über den Vakuumanschluss Vakuum angelegt. Anschließend wird ein Monomervorratsbehälter (beinhaltend die zweite Komponente/ein flüssiges Monomer) geöffnet. Das Monomer fließt infolge des Vakuums beziehungsweise der Schwerkraft durch das Rohr direkt in das Zementpulver (die erste Komponente). Nach erfolgtem Transfer des Monomers in das Zementpulver wird das Rohr mit dem Monomervorratsbehälter aus dem Inneren der Kartusche herausgezogen. Dann wird der Drehverschluss um wenige Grad weiter gedreht, so dass auch die Durchführungen für das Rohr im Kartuschenkopf als auch in der Porenscheibe so abgedichtet sind, dass anschließend unter Vakuum gemischt werden kann. Nach beendetem Mischprozess wird der Rührstab/Mischstab an den Mischkopf gezogen und der Verschluss im hohlen Rührstab/Mischstab herausgezogen. Dadurch ist das Austrägsrohr freigelegt und es kann der Zement durch das Austragsrohr ausgepresst werden.

Im Folgenden werden Ausführungsbeispiele der Erfindung anhand von acht schematisch dargestellten Figuren erläutert. Dabei zeigt:
- Figur 1:: eine schematische Querschnittansicht in Längsrichtung einer erfindungsgemäßen Mischvorrichtung;
- Figur 2:: eine schematische Querschnittansicht in Längsrichtung eines Teils einer erfindungsgemäßen Mischvorrichtung;
- Figur 3:: eine schematische Aufsicht auf eine erfindungsgemäße Mischvorrichtung;
- Figur 4:: eine schematische Aufsicht auf einen Verschluss einer erfindungsgemäßen Mischvorrichtung;
- Figur 5:: eine perspektivische Ansicht einer erfindungsgemäßen Mischvorrichtung;
- Figur 6:: eine schematische Ansicht eines Verschlusses für eine erfindungsgemäße Mischvorrichtung a) in Aufsicht, b) von unten und c) von der Seite;
- Figur 7:: eine schematische Querschnittansicht eines Einsatzes in den Kartuschenkopf einer erfindungsgemäße Mischvorrichtung; und
- Figur 8:: eine schematische Aufsicht auf Einsätze für erfindungsgemäße Mischvorrichtungen, und zwar a) oberer Teil und b) unterer Teil;

Alle beschriebenen erfindungsgemäßen Mischvorrichtungen und erfindungsgemäße Vakuumzementiersysteme sind zur Ausführung des erfindungsgemäßen Verfahrens geeignet.

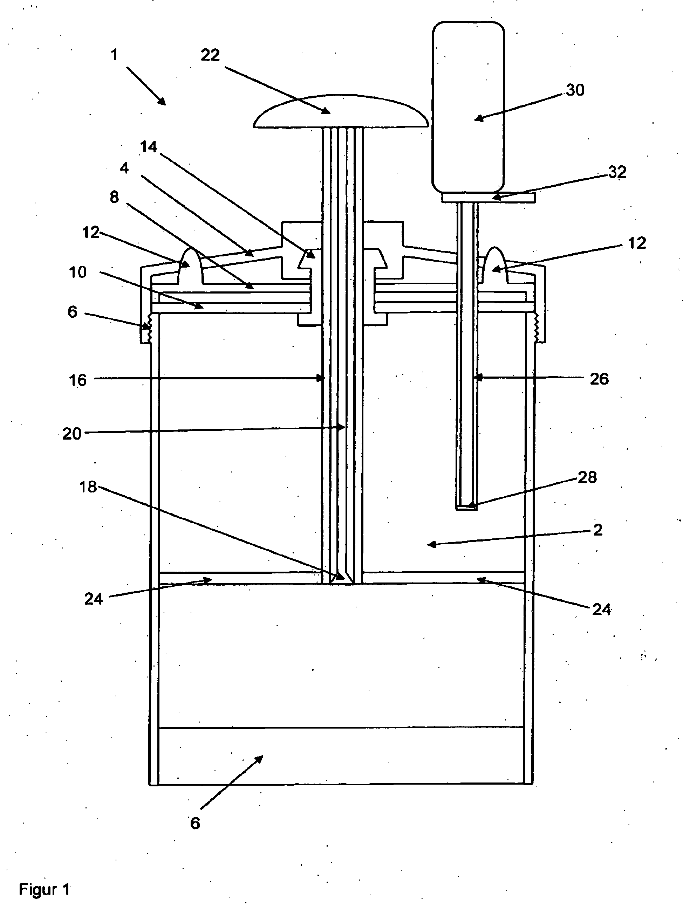

Figur 1 zeigt eine schematisch dargestellte Querschnittansicht einer erfindungsgemäßen Mischvorrichtung. Die Mischvorrichtung (1) umfasst eine Kartusche (2) mit einem Kartuschenkopf (4), der über ein Befestigungsmittel (6) in Form eines Gewindes mit dem Kartuschenkörper verbunden ist. Die Kartusche (2) Ist bodenseitig mit einem Förderkolben (6) verschlossen, der beweglich in Richtung des Kartuschenkopfs (4) angeordnet ist. Unterhalb des Kartuschenkopfs (4) sind ein drehbar gelagerter Verschluss (8) und darunter ein Porenfilter (10) zwischen dem Kartuschenkopf (4) und dem Inneren der Kartusche (2) angeordnet. Der Porenfilter (10) dient dazu, dass ein Pulver, das im Inneren der Kartusche (2) gelagert ist, nicht bis zum Verschluss (8) durchdringen kann, während Gase den Porenfilter (10) leicht passieren können.

Der Verschluss (8) umfasst zwei Griffe (12), die sich durch zwei Belüftungsöffnungen im Kartuschenkopf (4) erstrecken. Die Belüftungsöffnungen im Kartuschenkopf (4) sind größer als die Griffe (12) des Verschlusses (8). Durch Drehen des Verschlusses (8) können die Belüftungsöffnungen im Kartuschenkopf (4) geöffnet und geschlossen werden.

Im Zentrum des Kartuscherikopfs (4), des Verschlusses (8) und des Porenfilters (10) sind zentrische Öffnungen vorgesehen, in denen eine Führungshülse (14), die gleichzeitig als Halterung dient und eine Dichtung (nicht gezeigt) umfassen kann, angeordnet ist. Im Inneren der Führungshülse (14) ist eine Durchführung für ein Austragsrohr (16) vorgesehen, wobei sich das Austragsrohr (16) durch die zentrischen Öffnungen des Kartuschenkopfs (4), des Verschlusses (8) und des Porenfilters (10) ins Innere der Kartusche (2) erstreckt. Das Austragsrohr (16) ist in Richtung des Förderkolbens (6) durch einen Austragsrohrverschluss (18) verstopft. Der Austragsrohrverschluss (18) ist über eine Stange (20) mit einem Griffstück (22) verbunden. Das Griffstück (22) verschließt das Austragsrohr (16) an der Spitze außerhalb der Kartusche (2). Das Griffstück (22) ist so an der Spitze des Austragsrohrs (16) befestigt, dass durch eine Drehung des Griffstücks (22) auch das Austragsrohr (16) in der Kartusche (2) gedreht wird. Mit dem Austragsrohr (16) drehen sich dann auch zwei Mischflügel (24), die im Inneren der Kartusche (2) mit dem Austragsrohr (16) verbunden sind. Durch Lösen der Befestigung des Griffstücks (22) und Herausziehen des Griffstücks (22) mit der Stange (20) wird auch der Austragsrohrverschluss (18) aus dem Austragsrohr (16) entfernt und das Vakuumzementiersystem, das durch die Mischvorrichtung (1) gebildet wird, ist dann bereit zum Applizieren eines Zements aus dem Inneren der Kartusche (2).

Insbesondere bei zylindrischem Aufbau der Mischvorrichtung (1) oder Teilen der Mischvorrichtung (1), vorzugsweise der Kartusche (2) und des Kartuschenkopfs (4), ist sowohl das Austragsrohr (16) als auch der Verschluss (8) um die Symmetrieachse drehbar.

Durch eine nicht zentrische Durchführung durch den Kartuschenkopf (4), den Verschluss (8) und den Porenfilter (10) erstreckt sich ein Rohr (26) oder ein Schlauch ins Innere der Kartusche (2), bis in die erste Komponente, beispielsweise das Zementpulver. Die nicht zentrische Durchführung für das Rohr (26) im Verschluss (8) erstreckt sich entlang einer Kreisbogenlinie, so dass der Verschluss (8) von der geschlossenen zur offenen Position der Belüftungsöffnungen im Kartuschenkopf (4) überführt werden kann und umgekehrt, ohne das das Rohr (26) die Bewegung des Verschlusses (8) behindert.

Das offene Ende des Rohrs (26) im Inneren der Kartusche (2) ist durch einen Filter (28) abgedeckt, der nur gasförmige oder flüssige Substanzen passieren lässt, und/oder es ist ein Sieb angebracht. Am anderen Ende des Rohrs (26) ist ein Behälter (30) angeordnet, indem sich eine Flüssigkeit befindet, die zusammen mit dem Pulver in der Kartusche (2) einen Zement bildet. Zwischen dem Rohr (26) und dem Behälter (30) ist ein bedienbares Ventil (32) angeordnet. Dieses Ventil (32) ist zunächst geschlossen. Das Rohr (26) kann entlang seiner Symmetrieachse beweglich in den nicht zentrischen Durchführungen im Kartuschenkopf (4), dem Verschluss (8) und dem Porenfilter (10) angeordnet sein.

Die Mischvorrichtung (1) befindet sich zunächst im geöffneten Zustand, das heißt, dass die Belüftungsöffnungen im Kartuschenkopf (4) zunächst nicht durch den Verschluss (8) verdeckt sind. In diesem Zustand wird die Mischvorrichtung (1) zunächst sterilisiert, indem sie beispielsweise mit Ethylenoxid begast wird. Das Ethylenoxid tritt durch die Belüftungsöffnungen im Kartuschenkopf (4) und den Porenfilter (10) ins Innere der Kartusche (2) ein, so dass eine vollständige Sterilisierung möglich ist.

Als nächstes werden die Belüftungsöffnungen im Kartuschenkopf (4) manuell durch Drehen des Verschlusses (8) an den Griffen (12) geschlossen. Anschließend wird das Innere der Kartusche (2) über einen Vakuumanschluss (nicht gezeigt) evakuiert. Danach wird das Ventil (32) geöffnet, so dass sich der flüssige Inhalt des Behälters (30) durch das Rohr (26) und den Filter (28) ins Innere der Kartusche (2) ergießt. Das Rohr (26) wird vollständig oder nur ein Stück aus dem Inneren der Kartusche (2) herausgezogen. Anschließend wird der Verschluss (8) weiter gedreht, so dass die nicht zentrische Durchführung für das Rohr (26) im Kartuschenkopf (4) und in dem Porenfilter (10) geschlossen wird. Dabei kann das Rohr (26) durch die Drehung des Verschlusses (8) auch abgebrochen oder abgeschnitten werden. In einem weiteren Zwischenschritt kann das Innere der Kartusche (2) wieder evakuiert werden.

Durch Drehen des Griffstücks (22) wird das Austragsrohr (16) mit den Mischflügeln (24) gedreht und so die flüssige zweite Komponente aus dem Behälter (30) mit dem Zementpulver als erste Komponente in der Kartusche (2) gemischt. Dabei entsteht das Mischgut, beziehungsweise in diesem Fall der Zement. Das Austragsrohr (16) wird bis zur Führungshülse (14) aus dem Inneren der Kartusche (2) herausgezogen oder durch Ausüben eines Drucks auf den Förderkolben (6) herausgeschoben. Durch Entfernen des Griffstücks (22) mit dem Austragsrohrverschluss (18) wird die Mischvorrichtung (1) geöffnet. Anschließend kann der fertige Zement mit dem Förderkolben (6) durch das Austragsrohr (16) appliziert werden.

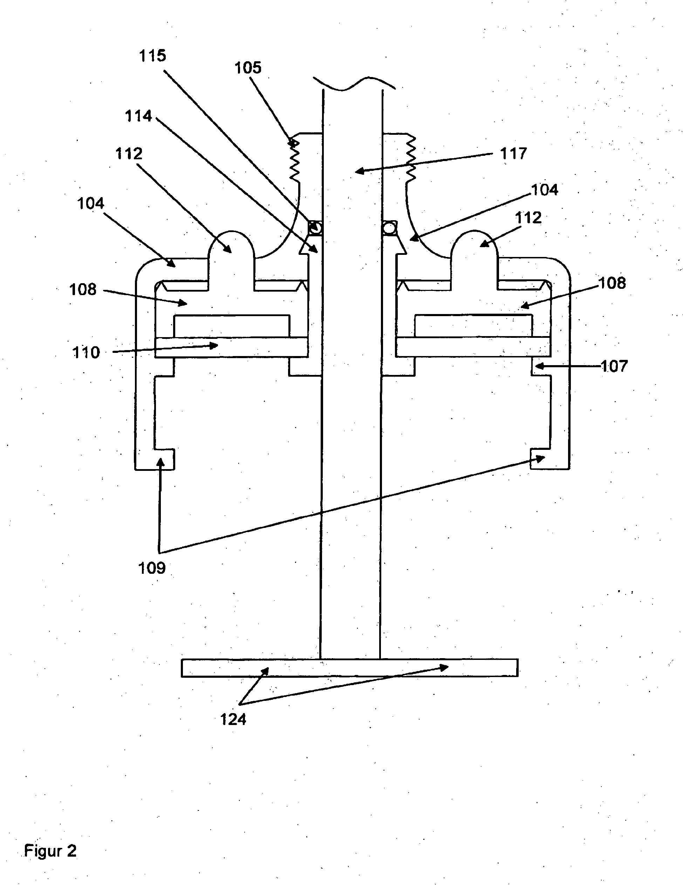

Figur 2 zeigt den schematischen Aufbau von Teilen einer alternativen. Mischvorrichtung in Querschnittansicht. In einem gezeigten Kartuschenkopf (104), der ein Gewinde (105) zum anschließen eines Austragsrohrs, zwei Belüftungsöffnungen und eine zentrische Öffnung sowie eine Halterung (107) umfasst, sind zwei verschiebbar gelagerte Verschlüsse (108) und ein als Ringscheibe ausgeformter Filter (110) in der Halterung (107) angeordnet. Durch die Belüftungsöffnungen erstrecken sich je ein Griff (112) der Verschlüsse (108). Die Belüftungsöffnungen im Kartuschenkopf (104) sind durch Verschieben der Verschlüsse (108) schließbar und zu öffnen. Am unteren Ende des Kartuschenkopfs (104) ist ein Befestigungsmittel in Form von Zapfen (109) angeordnet, die eine Befestigung des Kartuschenkopfs (104) an einen Kartuschenkörper (nicht gezeigt) ermöglichen.

In der zentrischen Öffnung ist eine Führungshülse (114) und ein Dichtungsring (115) angeordnet. Die Führungshülse (114) dient der Positionierung der Verschlüsse (108), des Filters (110) und des Dichtungsrings (115) sowie der Führung eines Mischstabs (117), der sich durch die zentrische Öffnung des Kartuschenkopfs (104) hindurch erstreckt. Am oberen Ende der Führungshülse (114) ist eine Schnappung zur Befestigung der Führungshülse (114) am Kartuschenkopf (104) vorgesehen. Der Dichtungsring (115) dient dazu, die zentrische Öffnung zusammen mit dem Mischstab (117) gasdicht abzuschließen. An dem Mischstab (117) sind zwei Mischflügel (124) aufgesteckt. Der Mischstab (117) kann durch die zentrische Öffnung herausgezogen werden, wobei die Mischflügel (124) abfallen oder weg klappen. Anschließend kann ein Austragsrohr auf dem Außengewinde (105) des Kartuschenkopfs (104) befestigt werden. Dazu hat das Austragrohr (nicht gezeigt) ein Innengewinde.

Der Mischstab (117) ist drehbar in der zentrischen Öffnung gelagert, so dass durch Drehen des Mischstabs (117) auch die Mischflügel (124) gedreht werden und die gezeigten Teile so einen Mischaufsatz nach Art eines Mixers für eine Kartusche (nicht gezeigt) bilden.

Als Filter (110) kann eine Porenscheibe verwendet werden. Anstatt zweier verschiebbarer Verschlüsse (108) kann auch ein einziger, drehbar gelagerter, durchbrochener Ring als Verschluss (108) verwendet werden. Die Durchbrechungen im ringförmigen Verschluss (108) überlagern die Belüftungsöffnungen im Kartuschenkopf (104), wenn sich der Verschluss (108) in der geöffneten Stellung befindet. Die hat den Vorteil, dass nur ein Verschluss (108) bewegt werden muss, um die Belüftungsöffnungen des Mischsystems zu schließen.

Oben an den verschiebbaren Verschlüssen (108) oder dem drehbaren Verschluss (108) sind Stege beziehungsweise als Stege ausgeformte Auflageringe angeordnet, die den Kontakt mit der Oberseite des Kartuschenkopfs (104) herstellen. Der dadurch hergestellte Abstand kann sicherstellen, dass ein Vakuumanschluss (nicht gezeigt) unabhängig von der Stellung des Verschlusses (108) immer geöffnet bleibt.

Werden diese Teile beziehungsweise ein solcher Einsatz an einer Kartusche, die mit einer ersten Komponente gefüllt ist und die mit einem zusätzlichen Behälter mit einer zweiten Komponente verbunden ist, verbunden, so ergibt sich eine erfindungsgemäße Mischvorrichtung.

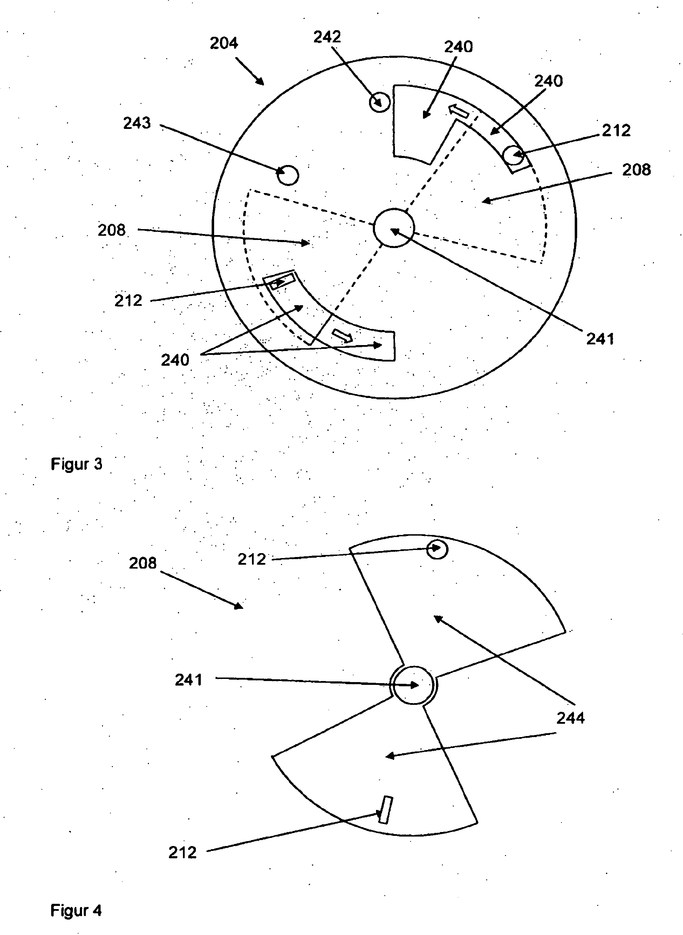

Figur 3 zeigt in schematischer Darstellung eine Aufsicht auf eine erfindungsgemäße Mischvorrichtung. In einem kreisrunden Kartuschenkopf (204) sind fünf Öffnungen vorgesehen, die teilweise durch einen unter dem Kartuschenkopf (204) angeordneten Verschluss (208) (in Figur 3 durch gestichelte Linien gekennzeichnet) verschließbar sind. Zwei der Öffnungen sind verschließbare Belüftungsöffnungen (240) in Form von Kreissektorabschnitten ("abgeschnittenen/angegessenen Tortenstücken"). Die nicht verschließbare zentrische Öffnung (241) dient der Durchführung eines Mischstabs und/oder eines Austragsrohrs ins Innere einer Kartusche, in dem sich eine erste pulverförmige Komponente zum Herstellen des Mischguts befindet. Eine verschließbare, nicht zentrische Durchführung (242) dient der Durchführung eines Rohrs, das an einen Behälter angeschlossen ist, in dem eine flüssige zweite Komponente zum Herstellen des Mischguts enthalten ist. Die letzte Öffnung ist ein nicht verschließbarer Vakuumanschluss (243), durch den das Innere der Kartusche evakuierbar ist. In dem Vakuumanschluss (243) kann ein Einwegventil (nicht gezeigt) angeordnet sein.

Der drehbar um die zentrische Öffnung (241) gelagerte Verschluss (208), der auch schematisch in Figur 4 gezeigt ist, umfasst zwei Blätter (244) in Form von Kreissektoren ("Tortenstücken"), die die Belüftungsöffnungen (240) und die nicht zentrische Durchführung (242) je nach Position des Verschlusses (208) abdecken können.

Befindet sich die Mischvorrichtung in einem, wie dem in Figur 3 gezeigten, offenen Zustand, sind die Belüftungsöffnungen.(240) und die nicht zentrische Durchführung (242) nicht durch den Verschluss (208) abgedeckt. Durch die Belüftungsöffnungen (240) kann das Innere der Kartusche mit einem Gas sterilisiert werden. Anschließend wird der Verschluss (208) in Richtung der nicht ausgefüllten Pfeile gedreht, so dass die Belüftungsöffnungen (240) geschlossen sind. Der Verschluss (208) kann nicht weiter gedreht werden, da in der nicht zentrischen Durchführung (242) ein Rohr steckt.

Anschließend wird die Mischvorrichtung über den Vakuumanschluss (243) evakuiert. Durch das Rohr in der nicht zentrischen Durchführung (242) wird eine flüssige zweite Komponente ins Innere der Kartusche geleitet beziehungsweise gesaugt und dort mit der ersten pulverförmigen Komponente durchmischt. Zur Durchmischung ist durch die zentrische Öffnung (241) ein Mischstab geführt, an dessen Ende einer oder mehrere Mischflügel angeordnet sind, so dass durch eine Drehung des Mischstabs eine Durchmischung der beiden Komponenten erfolgt.

Anschließend wird das Rohr aus der nicht zentrischen Durchführung (242) gezogen. Danach kann durch eine Weiterführung der Drehung des Verschlusses (208) auch die nicht zentrische Durchführung (242) durch den Verschluss (208) abgedeckt werden. Wenn der Mischstab hohl ist oder nach Entfernen des Mischstabs kann das fertige Mischgut durch die zentrische Öffnung (241) aus der Kartusche ausgetrieben werden.

Nach jedem Zwischenschritt, bei dem eine der Öffnungen (240, 242) verschlossen wird, kann es sinnvoll sein, das Innere der Kartusche über den Vakuumanschluss (243) zu evakuieren.

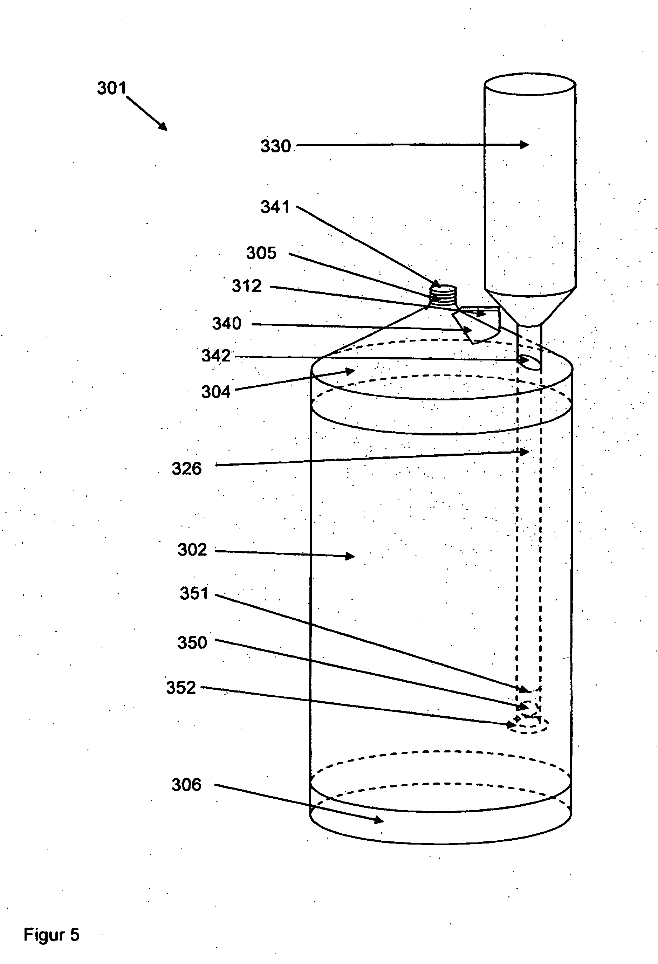

Figur 5 zeigt eine schematische Darstellung einer perspektivischen Ansicht einer erfindungsgemäßen Mischvorrichtung (301). Die Mischvorrichtung (301) umfasst eine Kartusche (302), die durch einen Kartuschenkopf (304) abgedeckt und druckdicht geschlossen ist. Im Kartuschenkopf (304) befindet sich eine Belüftungsöffnung (340), die durch einen drehbar oder verschiebbar gelagerten Verschluss (nicht gezeigt), der unterhalb des Kartuschenkopfs (304) angeordnet ist, verschließbar ist. Der Verschluss ist mit Hilfe eines Griffs (312) drehbar oder verschiebbar, wodurch die Belüftungsöffnung (340) zu öffnen und zu schließen ist. Der Griff (312) ragt durch die Belüftungsöffnung (340) nach außen.

In der Mitte des Kartuschenkopfs (304) befindet sich eine zentrische Öffnung (341), die durch ein Rohr gebildet ist, auf dessen Außenseite ein Gewinde (305) angeordnet ist. Durch eine nicht zentrische Durchführung (342) erstreckt sich ein Rohr (326), das mit einem Behälter (330) verbunden ist. Das Rohr (326) mündet in einem seitlich in der Rohrwand angeordneten Auslass (350) im Inneren der Kartusche (302). Das bodenseitige Ende des Rohrs (326) ist verschlossen. In der Kartusche (302) ist eine erste Komponente enthalten, während in dem Behälter (330) eine zweite Komporiente enthalten ist. Die zweite Komponente kann der ersten Komponente durch das Rohr (326) zugeführt werden. Im Bereich des Auslasses (350) ist eine Sollbruchstelle (351) und ein Stopper (352) in Form einer Scheibe am Rohr (326) angeordnet. Das Rohr (326) kann durch die nicht zentrische Durchführung (342) also nur bis zum Stopper (352) gezogen werden. Durch die seitliche Anordnung des Auslasses (350) ist dieser dann verschlossen. Das Rohr (326) kann dann einfach an der Sollbruchstelle (351) abgebrochen werden und die Misch-Vorrichtung (301) bleibt dicht abgeschlossen, während der leere Behälter (330) entfernt werden kann.

Durch die zentrische Öffnung (341) kann ein Mischstab (nicht gezeigt) ins Innere der Kartusche (302) eingeführt werden, um die beiden Komponenten im Inneren der Kartuschen (302) zu mischen. Auf das Gewinde (305) kann ein Austragsrohr (nicht gezeigt) aufgeschraubt werden.

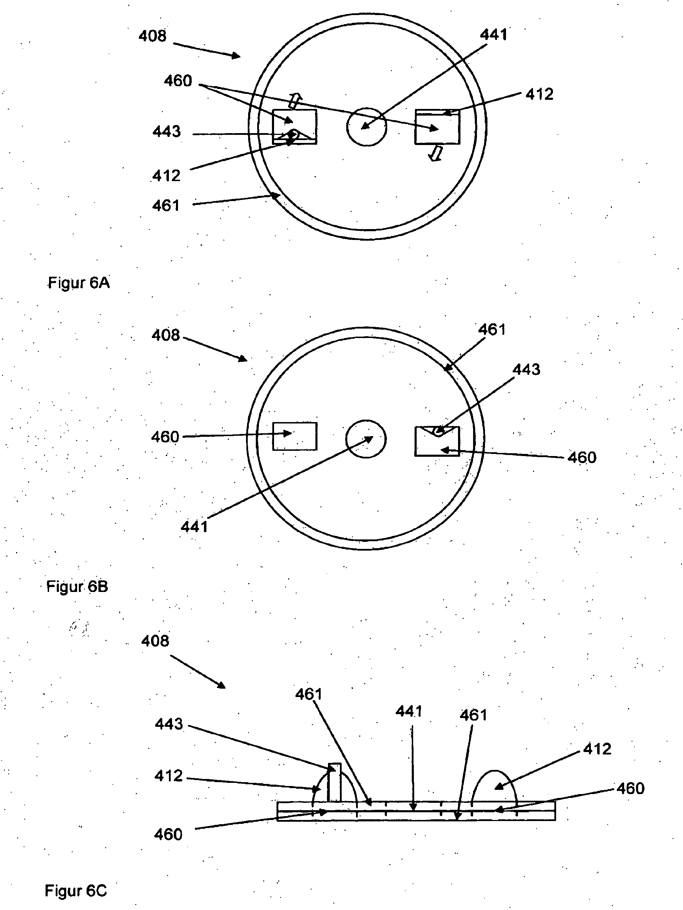

Figur 6 zeigt eine schematische Darstellung eines Verschlusses (408) für eine erfindungsgemäße Mischvorrichtung, der als drehbarer, durchbrochener Ring ausgebildet ist. Dabei zeigt Figur 6A eine Aufsicht (aus Richtung des Kartuschenkopfs), Figur 6B eine Ansicht von unten (aus Richtung des Kartuschenbodens) und Figur 6C eine Seitenansicht.

Der ringförmige Verschluss (408) hat in der Mitte eine zentrische Öffnung (441). Am Rand des Verschlusses (408) befindet sich auf der Oberseite und auf der Unterseite ein Auflagering (461). Der Verschluss (408) umfasst des Weiteren zwei Belüftungsöffnungen (460), mit denen eine durchgehende Belüftungsöffnung ins Innere einer erfindungsgemäßen Mischvorrichtung einstellbar ist, wenn die Belüftungsöffnungen (460) mit korrespondierenden Belüftungsöffnungen in einem Kartuschenkopf (nicht gezeigt) einer erfindungsgemäßen Mischvorrichtung überlappen.

Die Position des Verschlusses (408) kann durch eine Drehung desselben eingestellt werden. Dazu sind an dem Verschluss (408) im Bereich der Belüftungsöffnungen (460) zwei Griffe (412) vorgesehen. An einem der Griffe (412) ist ein Vakuumanschluss (443) angeordnet.

Ein solcher Verschluss (408) kann unterhalb oder auch oberhalb eines Kartuschenkopfs angeordnet werden und so zu einer erfindungsgemäßen Mischvorrichtung beitragen.

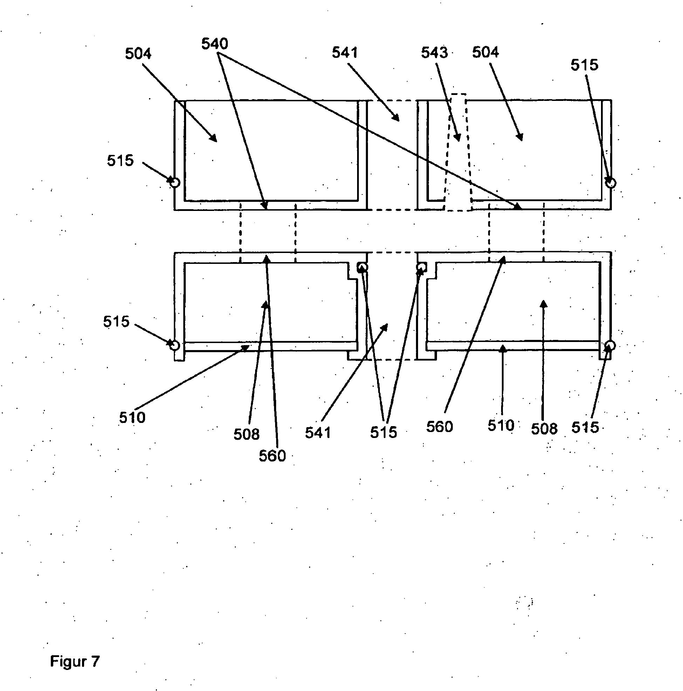

Figur 7 zeigt eine schematische Darstellung einer Querschnittansicht eines Kartuschenkopfs (504) mit einem Verschluss (508) und deren Zusammenwirken zur Ausbildung einer erfindungsgemäßen Mischvorrichtung. Sowohl der Kartuschenkopf (504) als auch der Verschluss (508) sind ringförmig aufgebaut und weisen an den Außenwänden Dichtungen (515) auf, die die Mischvorrichtung vakuumdicht und druckdicht abdichten, wenn der Kartuschenkopf (504) und der Verschluss (508) in eine hohlzylinderförmige Kartusche (nicht gezeigt) eingesetzt werden.

Auf der Innenseite des Verschlusses (508) ist eine weitere Dichtung (515) vorgesehen, um die Mischvorrichtung abzudichten, wenn ein Mischstab oder ein Austragsrohr in eine zentrische Öffnung (541) des Verschlusses (508) und des Kartuschenkopfs (504) eingesetzt wird.

In einer Halterung des Verschlusses (508) ist ein Porenfilter (510) angeordnet, der den Verschluss (508) bodenseitig vollständig abschließt. Auf der Oberseite des Verschlusses (508) sind zwei Belüftungsöffnungen (560) vorgesehen. Diese Belüftungsöffnungen (560) des Verschlusses (508) korrespondieren mit zwei Belüftungsöffnungen (540) des Kartuschenkopfs (504). Im Kartuschenkopf (504) ist.des Weiteren eine Vakuumanschluss (543) vorgesehen. In dem Vakuumanschluss (543) kann ein Ventil angeordnet sein, dass ausschließlich eine Strömung aus der Mischvorrichtung heraus (in Figur 7 nach oben) erlaubt.

Der Verschluss (508) ist drehbar relativ zum Kartuschenkopf (504) angeordnet. Wenn die Belüftungsöffnungen (540) des Kartuschenkopfs (504) die Belüftungsöffnungen (560) des Verschlusses (508) überdecken, existiert eine durchgehende Belüftungsöffnung. Durch Drehen des Verschlusses (508) oder des Kartuschenkopfs (504) wird diese Belüftungsöffnung geschlossen.

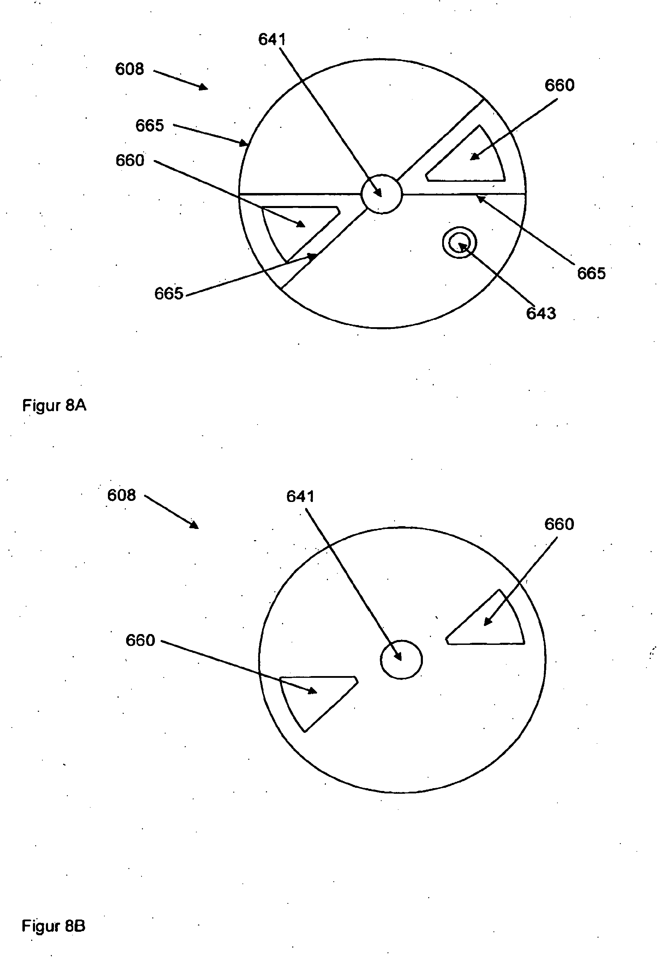

Die Figuren 8A und 8B zeigen schematische Darstellung eines erfindungsgemäßen Verschlusses (608) für eine erfindungsgemäße Mischvorrichtung von oben (Figur 8A) und von unten (Figur 8B). Eine zentrische Öffnung (641) ist im Zentrum des kreisrunden Verschlusses (608) vorgesehen. Zudem sind Belüftungsöffnungen (660) in Form von Kreissektoren im Verschluss (608) angeordnet. In der Oberseite des Verschlusses (608) ist zusätzlich ein Vakuumanschluss (643) vorgesehen.

Auf der Oberseite des Verschlusses (608) sind Stege (665) angeordnet, mit denen einen Abstand zu einem über dem Verschluss (608) angeordneten Kartuschenkopf (nicht gezeigt) gehalten wird. Die Stege (665) sind umlaufend am Rand des Verschlusses (608) und um die Belüftungsöffnungen (660) und die zentrische Öffnung (641) herum angeordnet.

### Bezugszeichenliste

- 1, 301: Mischvorrichtung
- 2, 302: Kartusche
- 4, 104, 204, 304, 504: Kartuschenkopf
- 105, 305: Gewinde
- 6, 306: Förderkolben
- 107: Halterung
- 8, 108, 208, 408, 508, 608: Verschluss
- 109: Zapfen
- 10,110,510: Porenfilter
- 12, 112, 212, 312, 412: Griff am Verschluss
- 14, 114: Führungshülse
- 115, 515: Dichtung
- 16: Austragrohr
- 117: Mischstab
- 18: Austragsrohrverschluss
- 20: Stange
- 22: Griffstück
- 24, 124: Mischflügel
- 26, 326: Rohr
- 28: Filter
- 30, 330: Behälter
- 32: Ventil
- 240, 340, 540: Belüftungsöffnung
- 241, 341, 441, 541, 641: zentrische Öffnung
- 242, 342: nicht zentrische Durchführung
- 243, 443, 543, 643: Vakuumanschluss
- 244: Blatt des Verschlusses
- 350: Auslass
- 351: Sollbruchstelle
- 352: Stopper
- 460, 560, 660: Belüftungsöffnung im Verschluss
- 461: Auflagering
- 665: Steg

## Patentansprüche

1. Mischvorrichtung (1, 301) zum Mischen eines Mischguts, insbesondere eines medizinischen Zements, umfassend zumindest eine Kartusche (2, 302) und einen Verschluss (8, 108, 208, 408, 508, 608), wobei die zumindest eine Kartusche (2, 302) eine erste Komponente des Mischguts beinhaltet und einen Kartuschenkopf (4, 104, 204, 304, 504) umfasst, in dem wenigstens eine Belüftungsöffnung (240, 340, 540) angeordnet ist und der Verschluss (8, 108, 208, 408, 508, 608) derart am Kartuschenkopf (4, 104, 204, 304, 504) angeordnet ist, dass die wenigstens eine Belüftungsöffnung (240, 340, 540) im Kartuschenkopf (4, 104, 204, 304, 504) durch den Verschluss (8, 108, 208, 408, 508, 608) verschließbar und zu öffnen ist, wobei
der Verschluss (8, 108, 208, 408, 508, 608) drehbar oder verschiebbar relativ zu dem Kartuschenkopf (4, 104, 204, 304, 504) gelagert ist und sich eine Verbindung (26, 326) durch den Kartuschenkopf (4, 104, 204, 304, 504) oder eine Kartuschenwand in das Innere der Kartusche (2, 302) erstreckt, die das Innere der Kartusche (2, 302) mit einem Behälter (30, 330) für eine zweite Komponente des Mischguts verbinden kann oder verbindet, wobei
zwischen der wenigstens einen Belüftungsöffnung (240, 340, 540) im Kartuschenkopf (4, 104, 204, 304, 504) und dem mit der ersten Komponente gefüllten Inneren der Kartusche (2, 302) ein Filter (10, 110, 510) und/oder ein Sieb angeordnet ist, dadurch ge- kennzeichnet, dass der Verschluss (8, 108, 208, 408, 508, 608) zwischen dem Kartuschenköpf (4, 104, 204, 304, 504) und dem Filter (10, 110, 510) oder dem Sieb angeordnet ist, wobei sich zumindest ein Teil (12, 112, 212, 312, 412) des Verschlusses (8, 108, 208, 408, 508, 608) in Kartuschenkopf (4, 104, 204, 304, 504) erstreckt.

2. Mischvorrichtung nach Anspruch 1, **dadurch gekennzeichnet, dass** die Verbindung (26, 326) ein Rohr (26, 326) oder ein Schlauch ist, wobei vorzugsweise zwischen der Verbindung (26, 326) und dem Behälter (30, 330) ein Ventil (32) angeordnet ist.

3. Mischvorrichtung nach Anspruch 1 oder 2, **dadurch gekennzeichnet, dass** die Verbindung (26, 326) in dem Kartuschenkopf (4, 104, 204, 304, 504) entlang ihrer Symmetrieachse verschiebbar ist.

4. Mischvorrichtung nach einem der vorangehenden Ansprüche, **dadurch gekennzeichnet, dass**
zwischen der wenigstens einen Belüftungsöffnung (240, 340, 540) im Kartuschenkopf (4, 104, 204, 304, 504) und dem mit der ersten Komponente gefüllten Inneren der Kartusche (2, 302) ein Porenfilter (10, 110, 510) angeordnet ist.

5. Mischvorrichtung nach einem der vorangehenden Ansprüche, **dadurch gekennzeichnet, dass**
die Mischvorrichtung (1, 301) ein Austragsrohr (16) oder eine Befestigungseinrichtung (105, 305) für ein Austragrohr (16) umfasst, wobei das Austragsrohr (16) vorzugsweise durch einen entfernbaren Austragsrohrverschluss (18) verschließbar ist

6. Mischvorrichtung nach einem der vorangehenden Ansprüche, **dadurch gekennzeichnet, dass**
die Verbindung (26, 326) einen seitlichen Auslass (350) im Bereich des Endes der Verbindung (26, 326) umfasst, das ins Innere der Kartusche (2, 302) reicht.

7. Mischvorrichtung nach einem der vorangehenden Ansprüche, **dadurch gekennzeichnet, dass**
die Verbindung (26, 326) zwischen ihrem Auslass (350) ins Innere der Kartusche (2, 302) und dem Behälter (30, 330) eine Sollbruchstelle (351) umfasst, vorzugsweise im Bereich des Auslasses (350).

8. Mischvorrichtung nach einem der vorangehenden Ansprüche, **dadurch gekennzeichnet, dass**
der Verschluss (8, 108, 208, 408, 508, 608) zwischen dem Kartuschenkopf (4, 104, 204, 304, 504) and dem Porenfilter (10, 110, 510) angeordnet ist, wobei sich wenigstens ein Griff (12, 112, 212, 312, 412) des Verschlusses (8, 108, 208, 408, 508, 608) durch die wenigstens eine Belüftungsöffnung (240, 340, 540) im Kartuschenkopf (4, 104, 204, 304, 504) erstreckt.

9. Mischvorrichtung nach einem der vorangehenden Ansprüche, **dadurch gekennzeichnet, dass**
zwei Belüftungsöffnungen (240, 340, 540) in dem Kartuschenkopf (4, 104, 204, 304, 504) angeordnet sind, die durch zwei Blätter (244) des Verschlusses (8, 108, 208, 408, 508, 608) verschließbar sind, wobei die Belüftungsöffnungen (240, 340, 540) und die Blätter (244) vorzugsweise als Kreissektoren ausgebildet sind und der Verschluss (8, 108, 208, 408, 508, 608) drehbar ist.

10. Mischvorrichtung nach einem der vorangehenden Ansprüche, **dadurch gekennzeichnet, dass**
am Verschluss (8, 108, 208, 408, 508, 608) zumindest ein Griff (12, 112, 212, 312, 412) angeordnet ist, vorzugsweise zwei Griffe (12, 112, 212, 312, 412) angeordnet sind, mit denen der Verschluss (8, 108, 208, 408, 508, 608) drehbar oder verschiebbar ist.

11. Mischvorrichtung nach einem der vorangehenden Ansprüche, **dadurch gekennzeichnet, dass**
ein Griffstück (22) mit dem Austragsrohr (16) oder einem Mischstab (117), der sich durch eine zentrische Öffnung (241, 341, 441, 541, 641) im Kartuschenkopf (4, 104, 204, 304, 504) von außen bis ins Innere der Kartusche (2, 302) erstreckt, verbunden oder verbindbar ist, mit dem das Austragsrohr (16) oder der Mischstab (117) im Inneren der Kartusche (2, 302) drehbar ist.

12. Mischvorrichtung nach Anspruch 11 und 5, **dadurch gekennzeichnet, dass**
der Austragsrohrverschluss (18) ein Stopfen (18) im Inneren der Austragsrohrs (16) ist, der mit dem Griffstück (22) verbunden ist, mit dem der Stopfen (18) aus dem Austragsrohr (16) entfernbar ist.

13. Mischvorrichtung nach einem der vorangehenden Ansprüche, **dadurch gekennzeichnet, dass**
am Austragsrohr (16) oder am Mischstab (117) wenigstens ein Mischflügel (24, 124) angeordnet ist und das Austragsrohr (16) oder der Mischstab (117) mit dem Mischflügel (24, 124) oder den Mischflügeln (24, 124) drehbar in der Kartusche (2, 302) gelagert ist.

14. Mischvorrichtung nach einem der vorangehenden Ansprüche, **dadurch gekennzeichnet, dass**
im Kartuschenkopf (4, 104, 204, 304, 504) oder in einer Wand der Kartusche (2, 302) ein Vakuumanschluss (243, 443, 543, 643) als Durchführung angeordnet ist, der vorzugsweise einen Ventilverschluss zum Öffnen und Schließen des Vakuumanschlusses (243, 443, 543, 643) umfasst.

15. Mischvorrichtung nach einem der vorangehenden Ansprüche, **dadurch gekennzeichnet, dass**
im Kartuschenboden ein Förderkolben (6, 306) zum Austreiben des Kartuscheninhalts angeordnet ist.

16. Mischvorrichtung nach einem der vorangehenden Ansprüche, **dadurch gekennzeichnet, dass**
die erste Komponente ein Pulver, insbesondere ein Zementpulver für einen PMMA-Knochenzement, ist, und die zweite Komponente eine Flüssigkeit, insbesondere eine Monomerflüssigkeit, ist.

17. Vakuumzementiersystem umfassend eine Mischvorrichtung (1, 301) nach einem der vorangehenden Ansprüche.

18. Verfahren zum Mischen eines Mischguts mit einer Mischvorrichtung (1, 301) nach einem der Ansprüche 1 bis 16 oder mit einem Vakuumzementiersystem nach Anspruch 17, mit den folgenden Verfahrensschritten.
A) das Innere und der Inhalt der Kartusche (2, 302) wird durch die wenigstens eine geöffnete Belüftungsöffnung (240, 340, 540) im Kartuschenkopf (4, 104, 204, 304, 504) sterilisiert, insbesondere unter Verwendung von Ethylenoxid,
B) die wenigstens eine Belüftungsöffnung (240, 340, 540) im Kartuschenkopf (4, 104, 204, 304, 504) wird durch Drehen oder Verschieben des Verschlusses (8, 108, 208, 408, 508, 608) relativ zum Kartuschenkopf (4, 104, 204, 304, 504) oder durch Drehen oder Verschieben des Kartuschenkopfs (4, 104, 204, 304, 504) relativ zum Verschluss (8, 108, 208, 408, 508, 608) geschlossen,
C) Gase aus dem Inneren der Kartusche (2, 302) werden evakuiert,
D) die zweite Komponente des Behälters (30, 330) wird in die Kartusche (2, 302) geleitet und nachdem die zweite Komponente ins Innere der Kartusche (2, 302) geleitet wurde, wird die Verbindung zum Behälter (30, 330) getrennt und der nicht zentrische Durchgang (242, 342) für die Verbindung zum Behälter (30, 330) wird durch Drehen oder Verschieben des Verschlusses (8, 108, 208, 408, 508, 608) relativ zum Kartuschenkopf (4, 104, 204, 304, 504) oder umgekehrt geschlossen und
E) die erste und die zweite Komponente werden gemischt.

19. Verfahren nach Anspruch 18, **dadurch gekennzeichnet, dass** die Mischung durch ein Austragsrohr (16) appliziert wird.

## Claims

1. A mixing device (1, 301) for mixing a mixture, in particular, a cement for medical use comprising at least one cartridge (2, 302) and a closure (8, 108, 208, 408, 508, 608), wherein the at least one cartridge (2, 302) includes a first component of the mixture and comprises a cartridge head (4, 104, 204, 304, 504), which has at least one air inlet (240, 340, 540), and the closure (8, 108, 208, 408, 508, 608) is arranged at the cartridge head (4, 104, 204, 304, 504) in such a way that the at least one air inlet (240, 340, 540) in the cartridge head (4, 104, 204, 304, 504) can be closed and opened by the closure (8, 108, 208, 408, 508, 608), wherein the closure (8, 108, 208, 408, 508, 608) is mounted rotatable or shiftable relative to the cartridge head (4, 104, 204, 304, 504) and a connection (26, 326) extends through the cartridge head (4, 104, 204, 304, 504) or a cartridge wall into the interior of the cartridge (2, 302), which connects or can connect the interior of the cartridge (2, 302) to a container (30, 330) for a second component of the mixture wherein a filter (10, 110, 510) and/or a sieve is located between the at least one air inlet (240, 340, 540) in the cartridge head (4, 104, 204, 304, 504) and the interior of the cartridge (2, 302) including the first component, **characterized in that**
the closure (8, 108, 208, 408, 508, 608) is located between the cartridge head (4, 104, 204, 304, 504) and the filter (10, 110, 510) and/or the sieve, wherein the at least one part (12, 112, 212, 312, 412) of the closure (8, 108, 208, 408, 508, 608) extends in the cartridge head (4, 104, 204, 304, 504).

2. The mixing device according to Claim 1, **characterized in that** the connection (26, 326) is a tube (26, 326) or a hose, wherein preferably, a valve (32) is located between the connection (26, 326) and the container (30, 330).

3. The mixing device according to Claim 1 or 2, **characterized in that** the connection (26, 326) is shiftable within the cartridge head (4, 104, 204, 304, 504) along its axis of symmetry.

4. The mixing device according to one of the preceding Claims, **characterized in that**
a pore filter (10, 110, 510) is located between the at least one air inlet (240, 340, 540) in the cartridge head (4, 104, 204, 304, 504) and the interior of the cartridge (2, 302) that is filled with the first component.

5. The mixing device according to one of the preceding Claims, **characterized in that**
the mixing device (1, 301) comprises a discharge tube (16) or a mounting device (105, 305) for a discharge tube (16), wherein the discharge tube (16) can preferably be closed by means of a removable discharge tube lock (18).

6. The mixing device according to one of the preceding Claims, **characterized in that**
the connection (26, 326) comprises a lateral outlet (350) in the area of the end of the connection (26, 326) extending into the interior of the cartridge (2, 302).

7. The mixing device according to one of the preceding Claims, **characterized in that**
the connection (26, 328) comprises a predetermined breaking point (351) between its outlet (350) into the interior of cartridge (2, 302) and the container (30, 330), preferably in the area of the outlet (350).

8. The mixing device according to one of the preceding Claims, **characterized in that**
the closure (8, 108, 208, 408, 508, 608) is located between the cartridge head (4, 104, 204, 304, 504) and the pore filter (10, 110, 510), wherein the at least one handle (12, 112, 212, 312, 412) of the closure (8, 108, 208, 408, 508, 608) extends through the at least one air inlet (240, 340, 540) in the cartridge head (4, 104, 204, 304, 504).

9. The mixing device according to one of the preceding Claims, **characterized in that**
two air inlets (240, 340, 540) are located in the cartridge head (4, 104, 204, 304, 504) that can be closed by means of two blades (244) of the closure (8, 108, 208, 408, 508, 608), wherein the air inlets (240, 340, 540) and the blades (244) are preferably designed as sectors and the closure (8, 108, 208, 408, 508, 608) is rotatable.

10. The mixing device according to one of the preceding Claims, **characterized in that**
at least one handle (12, 112, 212, 312, 412) is arranged at the closure (8, 108, 208, 408, 508, 608), preferably two handles (12, 112, 212, 312, 412), by means of which the closure (8, 108, 208, 408, 508, 608) can be rotated or shifted.

11. The mixing device according to one of the preceding Claims, **characterized in that**
a handle piece (22) is connected or can be connected with the discharge tube (16) or a mixing rod (117) that extends through a centric opening (241, 341, 441, 541, 641) in the cartridge head (4, 104, 204, 304, 504) from the outside into the interior of the cartridge (2, 302), by means of which the discharge tube (16) or the mixing rod (117) is rotatable in the interior of the cartridge (2, 302).

12. The mixing device according to Claim 11 and 5, **characterized in that**
the discharge tube lock (18) is a stopper (18) in the interior of the discharge tube (16), that is connected to the handle piece (22) by means of which the stopper (18) can be removed from the discharge tube (16).

13. The mixing device according to one of the preceding Claims, **characterized in that**
at least one mixing blade (24, 124) is arranged at the discharge tube (16) or at the mixing rod (117) and the discharge tube (16) or the mixing rod (117) is mounted rotatable in the cartridge (2, 302) together with the mixing blade (24, 124) or the mixing blades (24, 124).

14. The mixing device according to one of the preceding Claims, **characterized in that**
a vacuum connection (243, 443, 543, 643) is located in the cartridge head (4, 104, 204, 304, 504) or in a wall of the cartridge (2, 302) as feed-through, preferably comprising a valve closure for opening and closing the vacuum connection (243, 443, 543, 643).

15. The mixing device according to one of the preceding Claims, **characterized in that**
a delivery piston (6, 306) is located in the cartridge bottom for discharging the contents of the cartridge.

16. The mixing device according to one of the preceding Claims, **characterized in that**
the first component is a powder, in particular, a cement powder for a PMMA bone cement, and the second component is a liquid, in particular, a monomer liquid.

17. A vacuum cementing system comprising a mixing device (1, 301) according to one of the preceding Claims.

18. A method for mixing a mixture by means of a mixing device (1, 301) according to one of Claims 1 through 16 or by means of a vacuum cementing system according to Claim 17 having the following method steps,
A) the interior and the content of the cartridge (2, 302) is sterilized by means of the at least one opened air inlet (240, 340, 540) in the cartridge head (4, 104, 204, 304, 504), in particular by using ethylene oxide,
B) the at least one air inlet (240, 340, 540) in the cartridge head (4, 104, 204, 304, 504) is closed by rotating or shifting the closure (8, 108, 208, 408, 508, 608) relative to the cartridge head (4, 104, 204, 304, 504) or is closed by rotating or shifting the cartridge head (4, 104, 204, 304, 504) relative to the closure (8, 108, 208, 408, 508, 608),
C) gases from the interior of the cartridge (2, 302) are evacuated,
D) the second component of the container (30, 330) is conveyed into the cartridge (2, 302), and subsequent to conveying the second component into the interior of the cartridge (2, 302), the connection with the container (30, 330) is disconnected and the non-centric feed-through (242, 342) for the connection to the container (30, 330) is closed by rotating or shifting the closure (8, 108, 208, 408, 508, 608) relative to the cartridge head (4, 104, 204, 304, 504) or the reverse, and
E) the first and the second component are mixed.

19. The method according to Claim 18, **characterized in that** the mixture is applied through a discharge tube (16).

## Revendications

1. Dispositif de mélange (1, 301) pour le mélange d'un produit à mélanger, notamment un ciment médicinal, comprenant au moins une cartouche (2, 302) et un système de fermeture (8, 108, 208, 408, 508, 608), dans lequel l'au moins une cartouche (2, 302) contient un premier constituant du produit à mélanger et comprend une tête de cartouche (4, 104, 204, 304, 504), dans laquelle au moins un orifice d'aération (240, 340, 540) est agencé, et le système de fermeture (8, 108, 208, 408, 508, 608) est rapporté sur la tête de cartouche (4, 104, 204, 304, 504) de telle manière que l'au moins un orifice d'aération (240, 340, 540) dans la tête de cartouche (4, 104, 204, 304, 504) peut être fermé, et est à ouvrir, par le système de fermeture (8, 108, 208, 408, 508, 608), dans lequel
le système de fermeture (8, 108, 208, 408, 508, 608) est logé, en pouvant être mis en rotation ou en pouvant se déplacer par rapport à la tête de cartouche (4, 104, 204, 304, 504), et un système de liaison (26, 326) s'étend vers l'intérieur de la cartouche (2, 302) en traversant la tête de cartouche (4, 104, 204, 304, 504) ou une paroi de la cartouche, qui peut relier ou relie l'intérieur de la cartouche (2, 302) avec un récipient (30, 330) pour un deuxième constituant du produit à mélanger,
dans lequel un filtre (10, 110, 510) et/ou un tamis sont disposés entre l'au moins un orifice d'aération (240, 340, 540) dans la tête de cartouche (4, 104, 204, 304, 504) et l'intérieur de la cartouche (2, 302) rempli avec le premier constituant, **caractérisé en ce que**
le système de fermeture (8, 108, 208, 408, 508, 608) est agencé entre la tête de cartouche (4, 104, 204, 304, 504) et le filtre (10, 110, 510) ou le tamis, dans lequel au moins une partie (12, 112, 212, 312, 412) du système de fermeture (8, 108, 208, 408, 508, 608s'étend dans la tête de cartouche (4, 104, 204, 304, 504).

2. Dispositif de mélange selon la revendication 1, **caractérisé en ce que** le système de liaison (26, 326) est un tube (26, 326) ou un tuyau, dans lequel, de préférence, une soupape (32) est disposée entre le système de liaison (26, 326) et le récipient (30, 330).

3. Dispositif de mélange selon les revendications 1 ou 2, **caractérisé en ce que** le système de liaison (26, 326) peut être déplacé le long d'un axe de symétrie dans la tête de cartouche (4, 104, 204, 304, 504).

4. Dispositif de mélange selon l'une des revendications précédentes, **caractérisé en ce**
**qu'**un filtre poreux (10, 110, 510) est disposé entre l'au moins un orifice d'aération (240, 340, 540) dans la tête de cartouche (4, 104, 204, 304, 504) et l'intérieur de la cartouche (2, 302) rempli avec le premier constituant.

5. Dispositif de mélange selon l'une des revendications précédentes, **caractérisé en ce que**
le dispositif de mélange (1, 301) comprend un tube d'évacuation (16), ou un dispositif de fixation (105, 305) pour un tube d'évacuation (16), dans lequel le tube d'évacuation (16) peut être fermé de préférence par un système de fermeture de tube d'évacuation (18) amovible.

6. Dispositif de dosage selon l'une des revendications précédentes, **caractérisé en ce que**
le système de liaison (26, 326) comprend une sortie (350) latérale dans la zone de l'extrémité du système de liaison (26, 326) qui atteint l'intérieur de la cartouche (2, 302).

7. Dispositif de mélange selon l'une des revendications précédentes, **caractérisé en ce que**
le système de liaison (26, 326) comprend un point de rupture souhaitée (351) entre sa sortie (350) vers l'intérieur de la cartouche (2, 302) et le récipient (30, 330), de préférence dans la zone de la sortie (350).

8. Dispositif de mélange selon l'une des revendications précédentes, **caractérisé en ce que**
le système de fermeture (8, 108, 208, 408, 508, 608) est disposé entre la tête de cartouche (4, 104, 204, 304, 504) et le filtre poreux (10, 110, 510), dans lequel au moins une poignée (12, 112, 212, 312, 412) du système de fermeture (8, 108, 208, 408, 508, 608) s'étend à travers l'au moins un orifice d'aération (240, 340, 540) dans la tête de cartouche (4, 104, 204, 304, 504).

9. Dispositif de mélange selon l'une des revendications précédentes, **caractérisé en ce que**
deux orifices d'aération (240, 340, 540), qui peuvent êtres fermés par deux feuillets (244) du système de fermeture (8, 108, 208, 408, 508, 608), sont disposés dans la tête de cartouche (4, 104, 204, 304, 504), dans lequel les orifices d'aération (240, 340, 540) et les feuillets (244) sont de préférence conçus sous forme de secteurs circulaires, et le système de fermeture (8, 108, 208, 408, 508, 608) peut être mis en rotation.

10. Dispositif de mélange selon l'une des revendications précédentes, **caractérisé en ce**
**qu'**au moins une poignée (12, 112, 212, 312, 412) est agencée sur le système de fermeture (8, 108, 208, 408, 508, 608), de préférence, deux poignées (12, 112, 212, 312, 412) sont agencées, avec lesquelles le système de fermeture (8, 108, 208, 408, 508, 608) peut être mis en rotation ou être déplacé.

11. Dispositif de mélange selon l'une des revendications précédentes, **caractérisé en ce**
**qu'**une partie de poignée (22) est reliée ou peut être reliée avec le tube d'évacuation (16), ou une baguette de mélange (117), qui s'étend à travers un orifice central (241, 341, 441, 541, 641) dans la tête de cartouche (4, 104, 204, 304, 504) de l'extérieur jusqu'à l'intérieur de la cartouche (2, 302), avec laquelle le tube d'évacuation (16), ou la baguette de mélange (117), peut être mis(e) en rotation à l'intérieur de la cartouche (2, 302).

12. Dispositif de mélange selon les revendications 11 et 5, **caractérisé en ce que** le système de fermeture du tube d'évacuation (18) est un bouchon (18) à l'intérieur du tube d'évacuation (16), qui est relié avec la partie de poignée (22), avec laquelle le bouchon (18) peut être enlevé hors du tube d'évacuation (16).

13. Dispositif de mélange selon l'une des revendications précédentes, **caractérisé en ce**
**qu'**au moins une pale de mélange (24, 124) est rapportée sur le tube d'évacuation (16), ou sur la baguette de mélange (117), et le tube d'évacuation (16), ou la baguette de mélange (117), est logé(e) avec la pale de mélange (24, 124), ou les pales de mélange (24, 124), en pouvant être mis(e) en rotation dans la cartouche (2, 302).

14. Dispositif de mélange selon l'une des revendications précédentes, **caractérisé en ce**
**qu'**un raccordement au vide (243, 443, 543, 643), sous la forme d'un guide de passage, est disposé dans la tête de cartouche (4, 104, 204, 304, 504) ou dans une paroi de la cartouche (2, 302), qui comprend de préférence une fermeture par soupape pour l'ouverture et la fermeture du raccordement au vide (243, 443, 543, 643).

15. Dispositif de mélange selon l'une des revendications précédentes, **caractérisé en ce**
**qu'**un piston de transport (6, 306) est disposé dans le fond de la cartouche pour expulser le contenu de la cartouche.

16. Dispositif de mélange selon l'une des revendications précédentes, **caractérisé en ce que**
le premier constituant est une poudre, notamment une poudre de cimentation pour un ciment osseux à base de PMMA, et le deuxième constituant est un liquide, notamment un liquide à base d'un monomère.

17. Système de cimentation sous vide comprenant un dispositif de mélange (1, 301) selon l'une des revendications précédentes.

18. Procédé pour le mélange d'un produit à mélanger avec un dispositif de mélange (1, 301) selon l'une des revendications 1 à 16, ou avec un système de cimentation sous vide selon la revendication 17, avec les étapes de procédé suivantes :
A) l'intérieur et le contenu de la cartouche (2, 302) sont stérilisés, notamment moyennant l'emploi d'oxyde d'éthylène, par l'au moins un orifice d'aération (240, 340, 540) ouvert dans la tête de cartouche (4, 104, 204, 304, 504),
B) l'au moins un orifice d'aération (240, 340, 540) dans la tête de cartouche (4, 104, 204, 304, 504) est fermé par une rotation ou un déplacement du système de fermeture (8, 108, 208, 408, 508, 608) par rapport à la tête de cartouche (4, 104, 204, 304, 504), ou par une rotation ou un déplacement de la tête de cartouche (4, 104, 204, 304, 504) par rapport au système de fermeture (8, 108, 208, 408, 508, 608),
C) les gaz provenant de l'intérieur de la cartouche (2, 302) sont évacués,
D) le deuxième constituant du récipient (30, 330) est transféré dans la cartouche (2, 302) et lorsque le deuxième constituant a été transféré à l'intérieur de la cartouche (2, 302), le système de liaison vers le récipient (30, 330) est séparé et est fermé par une rotation ou un déplacement du système de fermeture (8, 108, 208, 408, 508, 608) par rapport à la tête de cartouche (4, 104, 204, 304, 504), ou inversement, et
E) le premier et le deuxième constituants sont mélangés.

19. Procédé selon la revendication 18, **caractérisé en ce que** le mélange est appliqué par un tube d'évacuation (16).
